(19) **Europäisches Patentamt**

**European Patent Office**

**Office européen des brevets**

(11) Publication number: **0 406 812 B1**

## EUROPEAN PATENT SPECIFICATION

(45) Date of publication of patent specification: **15.02.95**  (51) Int. Cl.6: **C07C 211/58**, C07C 209/62

(21) Application number: **90112703.5**

(22) Date of filing: **03.07.90**

(54) **Process for the preparation of NiNi-dialkyl-1,8-naphthalene diamine.**

(30) Priority: **03.07.89 US 375166**

(43) Date of publication of application:
**09.01.91 Bulletin 91/02**

(45) Publication of the grant of the patent:
**15.02.95 Bulletin 95/07**

(84) Designated Contracting States:
**DE FR GB**

(56) References cited:
**EP-A- 0 083 311      EP-A- 0 084 208**
**EP-A- 0 155 507      FR-A- 2 514 774**
**US-A- 4 122 027      US-A- 4 179 395**

**APPLIED PHYSICS LETTERS vol. 57, no. 23, 3 December 1990, pages 2399-2401, American Institute of Physics, US; V. S. WILLIAMS et al.: "Picosecond all-optical logic gate in a nonlinear organic étalon"**

**CHEMICAL ABSTRACTS vol. 70, no. 13, 31 March 1969, page 386, right-hand column, abstract no. 57920z, Columbus, Ohio, US; & JP - A - 68024180 (SHIONOGI) 18.12.1968**

(73) Proprietor: **HERCULES INCORPORATED**
**Hercules Plaza**
**Wilmington**
**Delaware 19894 (US)**

(72) Inventor: **Shannon, Paul Joseph**
**503 Balderston Drive**
**Exton,**
**Pennsylvania 19341 (US)**

(74) Representative: **Lederer, Franz, Dr. et al**
**Lederer, Keller & Riederer**
**Patentanwälte**
**Prinzregentenstrasse 16**
**D-80538 München (DE)**

CHEMICAL ABSTRACTS vol. 55, no. 15, 24 July 1961, right-hand column, abstract no. 14472f, Columbus, Ohio, US; N. VINOT.: "Synthesis of 2,3-dihydroperimidines substituted in the 2-position." & Compt. rend. 1961, vol. 252, pages 899-901

JOURNAL OF CHEMICAL SOCIETY Perkin I 1981, pages 2840-2847; R.W. ALDER et al.: "Preparation of a Range of NNN'N'-Tetrasubstituted 1,8-Diaminonaphathalenes"

CHEMISTRY OF HETEROCYCLIC COMPOUNDS 1975, pages 782-784, Plenum Press, New York, US; V.I. SOKOLOV et al.: "Heterocyclic analogs of Pleiadiene."

**Description**

This invention relates to a process for making N,N'-di($C_1$-$C_{20}$)alkyl-1,8-naphthalene diamine by hydrolyzing 2,3-dihydro-1,3-dialkyl-2,2-dialkyl perimidines.

J. Org. Chem. 51 (1986), pages 370-380 discloses 2,3-dihydro-1,3-dimethyl-2,2-dimethyl perimidine and 2,3-dihydro-1,3-dimethyl perimidine.

J.C.S. Perkin I (1981), pages 2840-47 describes a process for the preparation of the N,N'-dimethyl-1,8-naphthalene and the alkylation of 2,3-dihydro-2,2-dimethyl perimidine with 1,3-di-bromopropane. EP-A-0 083 311 describes the perimidine starting material.

Diazo dyes and pentaazo dyes containing a 2,3-dihydro-2,2-disubstituted perimidine substituent, in which the nitrogen atoms at the 1 and 3 positions of the dihydroperimidine are bound to a hydrogen atom, are known respectively from US-A-4,667,020 and US-A-4,122,027.

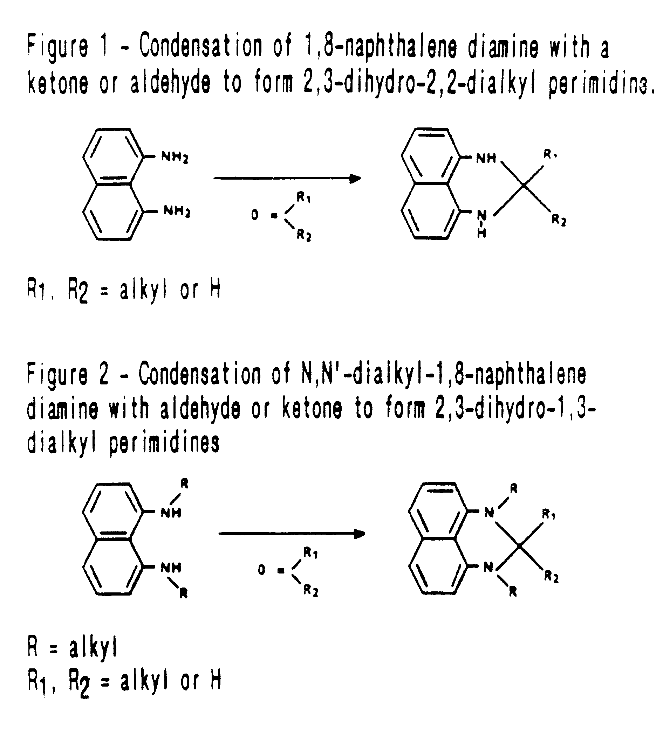

These U.S. Patents disclose making 2,3-dihydro-2,2-disubstituted perimidines by condensing 1,8-naphthalene diamine with a ketone or aldehyde as outlined in Figure 1.

Because the N-H bonds in such dyes result in relatively high crystalline melting points, azo dyes containing dihydroperimidine substituents unsubstituted at the nitrogen positions have relatively low solubility levels in nematic hosts.

A process for making 2,3-dihydro perimidines includes the step of condensation of a 1,8-naphthalene diamine with a ketone or aldehyde, whereby an N,N'-dialkyl-1,8-naphthalene diamine is condensed with a ketone or aldehyde to produce a 2-substituted or 2,2-disubstituted 2,3-dihydro-1,3-dialkyl-perimidine, as outlined in Figure 2.

Preferably, the condensation is carried out in a solvent at room temperature in the presence of a catalytic amount of p-toluene sulfonic acid. The solvent may be the aldehyde or ketone if appropriate, for instance if the ketone is acetone, or it may be an inert solvent such as tetrahydrofuran or ethyl acetate, in which case a slight excess of the aldehyde or ketone is desirable.

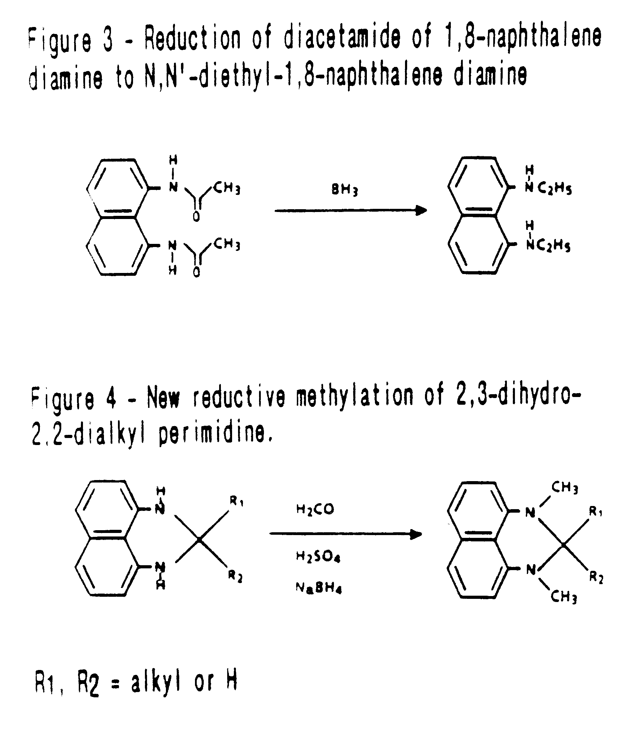

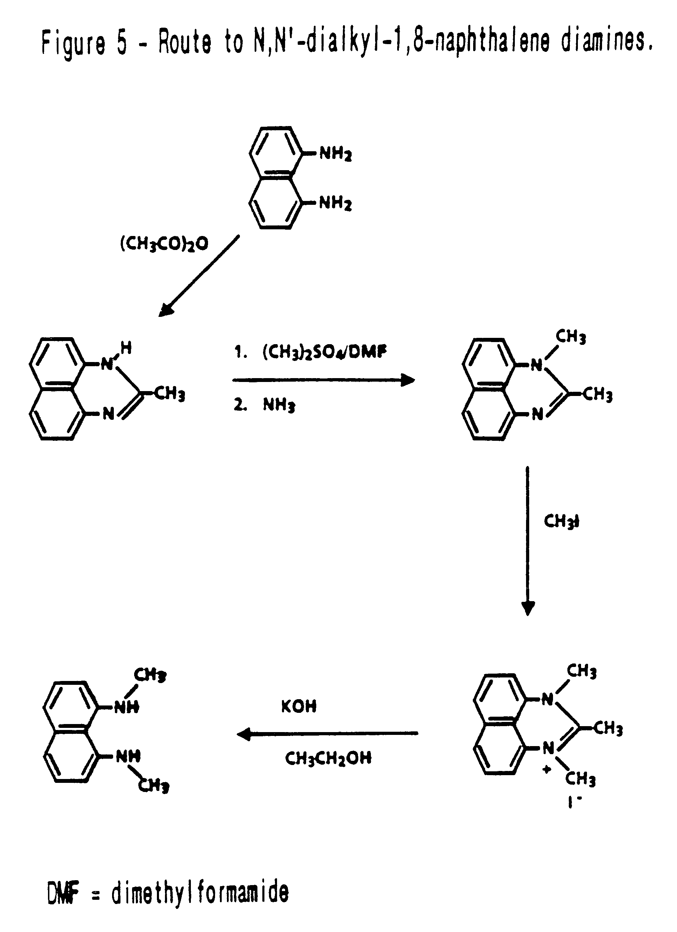

The preparation of N,N'-dialkyl-1,8-naphthalene diamines by steps comprising condensation of 1,8-naphthalene diamine with acetic anhydride to give 2-methyl perimidine, alkylation of the perimidine with dimethyl sulfate and basification with ammonia to give 1,2-dimethyl perimidine, alkylation with methyl iodide and hydrolysis of the methiodide salt (as outlined in Figure 5) is known from A. F. Pozharskii et al, Zh. Org. Khim., 16, (10) 2216 (1980) and Chemistry of Heterocyclic Compounds, Plenum Press, New York p 782 (1975) (Translated from Khim. Geterot. Soed., No. 6, 849 (1973)) to give the desired N,N'-dimethyl-1,8-naphthalene diamine. Another method of preparation (as outlined in Figure 3) requires the reduction of the diacetamide of 1,8-naphthalene diamine and is disclosed by R. W. Alder, et al, J. Chem. Soc., Pt. 1, 2840 (1981). These known methods of forming N,N'-dialkyl-1,8-naphthalene diamines are not generally satisfactory or applicable at all for making many desirable N,N'-dialkyl-1,8-naphthalene diamines.

Preferably therefore the N,N'-dialkyl-1,8-naphthalene diamine is prepared by a novel process comprising the steps of condensing a 1,8-naphthalene diamine with a ketone or aldehyde, preferably a ketone, to produce a 2,3-dihydro-2,2-dialkyl perimidine or 2,3-dihydro-2-alkyl perimidine, alkylating the 2,3-dihydro-2,2-dialkyl perimidine at each nitrogen atom or 2,3-dihydro-2-alkyl perimidine at the nitrogen atom with an alkyl halide, preferably a bromide or iodide, alkyl tosylate or alkyl mesylate alkylating agent, more preferably methyl iodide, butyl iodide, methyl bromide, octyl bromide, methyl tosylate, octyl tosylate, methyl mesylate, or decyl mesylate, in a water miscible organic solvent, preferably acetone, methyl ethyl ketone, tetrahydrofuran or dimethylformamide, more preferably acetone and methyl ethyl ketone, in the presence of water and an inorganic base, preferably potassium carbonate, sodium carbonate, sodium hydroxide, potassium hydroxide, more preferably potassium carbonate, to produce a 2,3-dihydro-1,3-dialkyl-2,2-dialkyl perimidine or 2,3-dihydro-1,3-dialkyl-2-alkyl perimidine, and hydrolysing, preferably at 60-100°C, the 2,3-dihydro-1,3-dialkyl-2,2-dialkyl perimidine or 2,3-dihydro-1,3-dialkyl-2-alkyl perimidine in the presence of a strong aqueous acid and in a water-miscible non-ketone organic solvent to produce the N,N'-dialkyl-1,8-naphthalene diamine.

Preferably the condensation step is carried out at room temperature and the alkylation step at a temperature between room temperature and 80°C. Preferably the aqueous acid is hydrochloric acid, sulfuric acid, para-toluene sulfonic acid, more preferably hydrochloric acid, and the water-miscible. non-ketone solvent is tetrahydrofuran, ethanol, methanol, more preferably tetrahydrofuran in water. Since the hydrolysis requires the removal of the ketone or aldehyde by-product as it is formed, the aqueous organic solvent is distilled during the reaction to drive over the ketone or aldehyde by-product as it is formed.

The steric hindrance about the nitrogen atoms of the 2,3-dihydro-2,2-dialkyl perimidines or 2,3-dihydro-2-alkyl perimidines controls the selectivity of the alkylation reaction. With methyl iodide, methyl tosylate, methyl mesylate, methyl bromide and the like high steric hindrance and low temperatures (e.g., room temperature) are preferred. With higher homologs, such as n-butyl iodide, octyl bromide. etc., less steric hindrance and higher temperatures (e.g., 60-80°C) are preferred.

2,3-Dihydro perimidines derived from acetone are very resistant to hydrolysis. All the solvent and much off the aqueous acid must be distilled to drive the hydrolysis to completion. 2,3-Dihydro perimidines derived from 3-methyl-2-butanone are hydrolyzed very readily. The reaction is usually complete when all the organic solvent has been distilled.

The N,N'-dialkyl-1,8-naphthalene diamines are easily purified by recrystallization. They can then be used to prepare a wide variety 2-substituted or 2,2-disubstituted 2,3-dihydro-1,3-dialkyl-perimidines by condensation with aldehydes or ketones according to the invention and as outlined in Figure 2. Condensations are preferably carried out at room temperature in the presence of a catalytic amount of p-toluene sulfonic acid. The aldehyde or ketone can be used as solvent, as in the case of the acetone adduct, or a slight excess of the aldehyde or ketone can be used in an inert solvent such as tetrahydrofuran or ethyl acetate. Table 2 lists preferred 2,3-dihydro-1,3-dialkyl perimidines prepared by condensation of aldehydes or ketones with N,N'-dialkyl-1,8-naphthalene diamines.

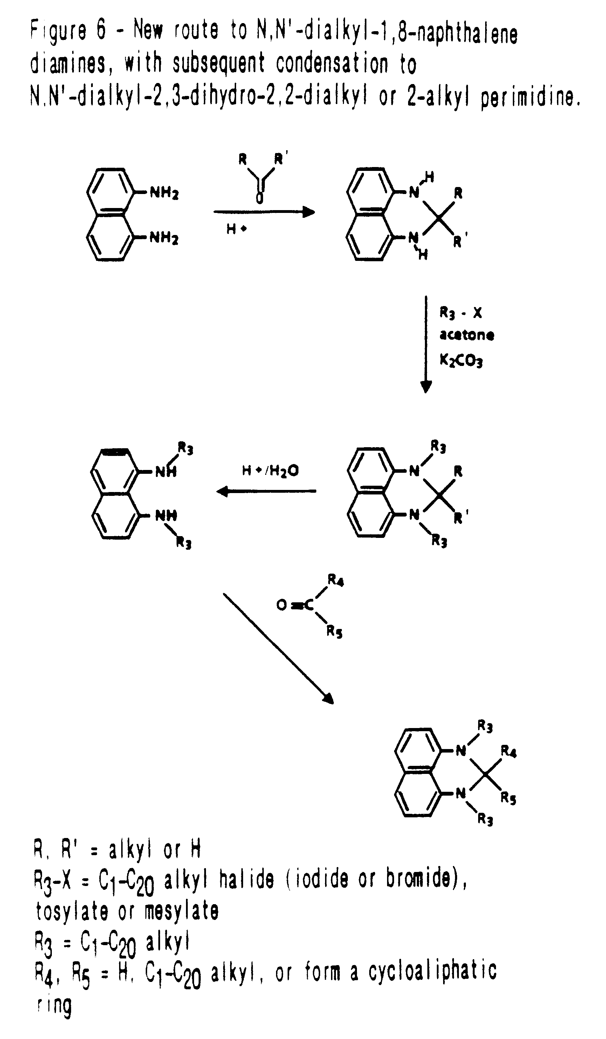

The said preferred process readily and economically prepares high yields of N,N'-dialkyl-1,8-naphthalene diamines that are suitable for use in the process for making 2,3-dihydro perimidines. Table 1 (Following Examples 1 - 3) lists preferred N,N'-dialkyl-1,8-naphthalene diamines prepared by the route outlined in Figure 6 and described above.

An alternative, less preferred, method for making 2,3-dihydro-1,3-dialkyl perimidines is reductive methylation of 2,3-dihydro perimidines with sodium borohydride and formaldehyde in aqueous sulfuric acid as outlined in Figure 4.

Also the 2,3-dihydro-1,3-dialkyl perimidines may be used in dichroic dyes having a 2,3-dihydro-1,3-dialkyl perimidine substituent.

A conventional synthetic scheme for preparing dichroic dyes, known for instance from US-A-4,122,027, involves direct diazotization of the appropriate 4-substituted aniline in strong mineral acid, followed by coupling the resultant diazo compound to the appropriate substituted aniline. Diazotization and coupling are repeated as desired and the intermediate products are recovered from a buffered aqueous solution as the free base and purified by recrystallization. The free base is subsequently diazotized and coupled with the desired substituted 2,3-dihydro perimidine in acid, which uses the 2,3-dihydro-1,3-dialkyl perimidines. The resultant dichroic dye is recovered from the neutralized solution by filtration and is purified by recrystallization or chromatography.

Example 1

This example illustrates the preparation of a N,N'-dimethyl-1,8-naphthalene diamine shown in Table 1 using the procedure of Figure 6.

1,8-Naphthalene diamine was distilled on a Kugelrohr distillation apparatus (Aldrich Chemical Co.) at 140-160°C (0.1 mm Hg) to give a pink solid. Then, a mixture of the distilled 1,8-naphthalene diamine (21.8 g, 0.138 mol, Molecular Weight 158), p-toluene sulfonic acid (0.75 g), and 3-methyl-2-butanone (24.6 g, 0.30 mol, molecular weight 82) was prepared, stirred at 50 - 60°C for 1 hour, and allowed to sit overnight at room temperature. The mixture was transferred to a 500 ml 3-neck flask. Water (30 ml), acetone (150 ml), potassium carbonate (84 g, 0.6 mol), and iodomethane (84 g, 0.60 mol) were added and the mixture mechanically stirred at 60 - 70°C for 2 hours, after which more iodomethane (21.0 g, 0.15 mol) was added. The reaction was monitored by thin layer chromatography (TLC) using hexane-ethyl acetate (5:1) as an eluting solvent. After 5.5 hours, the excess methyl iodide and some acetone (25 ml) was distilled and the mixture diluted with water (500 ml) and extracted with ether (2 times. 400 ml and 200 ml portions). The ether extracts were washed with brine and concentrated to a black solid. The solid was dissolved in distilled tetrahydrofuran (THF) (100 ml) and 2 N HCl (in distilled water) (300 ml). The THF and 100 ml of water were distilled. TLC indicated that the hydrolysis reaction was complete. The mixture was cooled, basified to a pH greater than 11 with ammonium hydroxide (150 ml) and extracted with ether (400 ml and 200 ml portions). The extract was washed with brine, dried over potassium carbonate and concentrated to a solid. The solid was distilled (Kugelrohr, 98-102°C, at 0.1 mm Hg) to give a beige solid (21.4 g). The solid was dissolved in hot ethyl acetate (40 ml) and diluted with hexane (160 ml). Cooling gave crystals of N,N'-dimethyl-1,8-naphthalene diamine (15.1 g, 59 %): melting point (mp) 102-104°C; NMR (CDCl$_3$) 7.15(m, 4H), 7.5(d of d, 2H), 5.4(bs, 2H), 2.85(s, 6H); IR (KBr) 3360, 1595 cm$^{-1}$.

Example 2

This example illustrates the synthesis of a N,N'-dibutyl-1,8-naphthalene diamine shown in Table 1 using the procedure of Figure 6.

A mixture of distilled 1,8-naphthalene diamine (0.14 mol) (distilled as described in Example 1), 3-methyl-2-butanone (24.6 g, 0.28 mol) and p-toluene sulfonic acid (0.75 g) was prepared and stirred at room temperature for 1 hour, and, then, allowed to sit overnight. Acetone (150 ml), water (30 ml), potassium carbonate (84 g, 0.61 mol), and n-butyl iodide (110 g, 0.60 mol) were added to the mixture, the mixture was heated to 60-70°C, and stirred at that temperature for 25 hours. Potassium carbonate (42.0 g, 0.305 mol) and n-butyl iodide (55 g, 0.30 mol) were added, the mixture was heated to 60-70°C, and stirred at that temperature for 19 hours. The mixture was diluted with water (750 ml) and extracted with ether (600 ml and 300 ml portions). The extract was washed with brine, dried over potassium carbonate, and concentrated. The mixture was heated in a Kugelrohr apparatus to 60°C at 0.1 mm Hg to remove 1-butanol. The remaining mixture, comprising the dialkylated, monoalkylated and nonalkylated product (by TIC analysis) was purified by column chromatography on silica (hexane-ethyl acetate, 20:1) to give predominately the desired dialkylated product (20.7 g). The material was dissolved in THF (100 ml) and 2 N HCl (300 ml) and heated to distill THF (100 ml). The mixture was basified with ammonium hydroxide, and extracted with ether (400 ml and 200 ml portions). The extract was washed with brine, dried over potassium carbonate, and concentrated. The residue was distilled on a Kugelrohr apparatus up to 120°C (0.1 mm Hg) to give a beige solid (12.8 g). Recrystallization from ethyl acetate-hexane (3:2) gave N,N'-dibutyl-1,8-naphthalene diamine as needles (5.4 g, 14 %): mp 63-64°C; NMR (CDCl$_3$) 7.1(m, 4H), 6.4(m, 2H), 5.35(bs, 2H), 3.02(t, 4H), 1.8-1.3(m, 8H), 0.95(t, 6H),; IR (KBr) 3330, 1595 cm$^{-1}$.

Example 3

This example illustrates the preparation of a N,N'dioctyl-1,8-naphthalene diamine shown in Table 1 using the procedure of Figure 6.

A mixture of distilled 1,8-naphthalene diamine (15.8 g, 0.10 mol) (distilled as described in Example 1), acetone (100 ml) and p-toluene sulfonic acid (0.50 g) was prepared and stirred at room temperature for 15 minutes. Potassium carbonate (55.0 g, 0.40 mol), water (20 ml) and n-octyl iodide (96 g, 0.40 mol) were added to the mixture, and the mixture heated to 65-70°C and stirred for 24 hours. Potassium carbonate (27.5 g, 0.20 mol) and n-octyl iodide (48 g, 0.20 mol) were added and the heating continued for another 24 hours. The mixture was diluted with water and extracted two times with 500 ml portions of ether. The ether extract was washed with water and brine, dried over potassium carbonate, and concentrated. The mixture was heated in a Kugelrohr apparatus to 80°C at 0.1 mm Hg to remove excess n-octyl iodide. The remaining material in the pot was purified by chromatography to give 14.5 g of an oil. The oil was dissolved in THF (200 ml) and 2 N hydrochloric acid (200 ml) and heated to distill all the THF and 100 ml of the aqueous acid. The mixture was basified with ammonium hydroxide, and extracted with ether (500 ml and 300 ml portions). The extract was washed with brine, dried over potassium, and concentrated. The residue was purified by chromatography with hexane-ether 20:1 to give a tinted orange solid (2.0 g): mp 42-43°C; NMR (CDCl$_3$) 7.1 (m, 4H), 6.45(m, 2H), 5.42(bs, 2H), 3.05(t, 4H), 1.8-1.3(m, 24H), 0.95(t, 6H); IR (CHCl$_3$) 3330, 1598 cm$^{-1}$.

EP 0 406 812 B1

## Table 1 - N,N'-Dialkyl-1,8-Napththalene Diamines of Examples 1-3

| Example No. | $R_3$ | Melting Point (°C) |
|---|---|---|
| 1 | $CH_3$ | 101.5–103.5 |
| 2 | $C_4H_9$ | 63–64 |
| 3 | $C_8H_{17}$ | 42–43 |

**Claims**

1. A process for preparing N,N'-di($C_1$-$C_{20}$)alkyl-1,8-naphthalene diamine comprising hydrolyzing of 2,3-dihydro-1,3-di($C_1$-$C_{20}$)alkyl-2,2-dialkyl perimidine with strong aqueous acid in an aqueous organic solvent and removing a ketone by-product and the aqueous organic solvent by co-distillation.

2. A process as claimed in claim 1 wherein the strong acid is selected from the group consisting of hydrochloric acid, sulfuric acid and p-toluenesulfonic acid.

3. A process for preparing N,N'-di($C_1$-$C_{20}$)alkyl-1,8-naphthalene diamine comprising:
   (a) alkylating 2,3-dihydro-2,2-dialkyl perimidine with an ($C_1$-$C_{20}$)-alkylating agent in a water miscible organic solvent at room temperature to 80°C in the presence of water and an inorganic base to form 2,3-dihydro-1,3-di($C_1$-$C_{20}$)alkyl-2,2-dialkyl perimidine; and
   (b) hydrolizing the 2,3-dihydro-1,3-di($C_1$-$C_{20}$)alkyl-2,2-dialkyl perimidine with strong aqueous acid in an aqueous organic solvent and removing a ketone by-product and the aqueous organic solvent by co-distillation.

4. A process as claimed in claim 3 wherein:
   (i) the alkylating agent has the formula R-X wherein R is $C_1$-$C_{10}$ alkyl and X is Br,I, tosylate group or mesylate group;
   (ii) the water miscible organic solvent is a ketone; and
   (iii) the inorganic base is selected from the group consisting of potassium carbonate, sodium carbonate, sodium hydroxide and potassium hydroxide.

5. A process as claimed in claim 3 wherein the strong acid is selected from the group consisting of hydrochloric acid, sulfuric acid and p-toluenesulfonic acid.

6. A process as claimed in claim 4 wherein the strong acid is selected from the group consisting of hydrochloric acid, sulfuric acid and p-toluenesulfonic acid.

7. A process as claimed in claim 3, further comprising condensation of 1,8-naphthalene diamine with a ketone to obtain 2,3-dihydro-2,2-dialkyl perimidine.

8. A process as claimed in claim 7 wherein:
   (i) the alkylating agent has the formula R-X wherein R is $C_1$-$C_{10}$ alkyl and X is Br,I, tosylate group or mesylate group;
   (ii) the water miscible organic solvent is a ketone; and

(iii) the inorganic base is selected from the group consisting of potassium carbonate, sodium carbonate, sodium hydroxide and potassium hydroxide.

9.  A process as claimed in claim 7 wherein the strong acid is selected from the group consisting of hydrochloric acid, sulfuric acid and p-toluenesulfonic acid.

10. A process as claimed in claim 8 wherein the strong acid is selected from the group consisting of hydrochloric acid, sulfuric acid and p-toluenesulfonic acid.

**Patentansprüche**

1.  Verfahren zur Herstellung von N,N'-Di($C_{1-20}$)alkyl-1,8-naphthalindiamin, umfassend das Hydrolysieren von 2,3-Dihydro-1,3-di($C_{1-20}$)alkyl-2,2-dialkylperimidin mit starker wässeriger Säure in einem wässerigen organischen Lösungsmittel und Entfernen eines Keton-Nebenprodukts und des wässerigen organischen Lösungsmittels durch Codestillation.

2.  Verfahren nach Anspruch 1, worin die starke Säure aus der Gruppe bestehend aus Chlorwasserstoffsäure, Schwefelsäure und p-Toluolsulfonsäure ausgewählt ist.

3.  Verfahren zur Herstellung von N,N'-Di($C_{1-20}$)alkyl-1,8-naphthalindiamin, welches umfaßt:
    (a) das Alkylieren von 2,3-Dihydro-2,2-dialkylperimidin mit einem ($C_{1-20}$)-Alkylierungsmittel in einem wassermischbaren organischen Lösungsmittel bei Raumtemperatur bis 80° C in Gegenwart von Wasser und einer anorganischen Base unter Bildung von 2,3-Dihydro-1,3-di($C_{1-20}$)alkyl-2,2-dialkylperimidin, und
    (b) Hydrolysieren des 2,3-Dihydro-1,3-di($C_{1-20}$)alkyl-2,2-dialkylperimidins mit starker wässeriger Säure in einem wässerigen organischen Lösungsmittel und Entfernen eines Keton-Nebenprodukts und des wässerigen organischen Lösungsmittels durch Codestillation.

4.  Verfahren nach Anspruch 3, worin:
    (i) das Alkylierungsmittel die Formel R-X aufweist, worin R $C_{1-10}$-Alkyl und X Br, I, eine Tosylatgruppe oder eine Mesylatgruppe bedeutet,
    (ii) das wassermischbare organische Lösungsmittel ein Keton ist, und
    (iii) die anorganische Base aus der Gruppe bestehend aus Kaliumcarbonat, Natriumcarbonat, Natriumhydroxid und Kaliumhydroxid ausgewählt ist.

5.  Verfahren nach Anspruch 3, worin die starke Säure aus der Gruppe bestehend aus Chlorwasserstoffsäure, Schwefelsäure und p-Toluolsulfonsäure ausgewählt ist.

6.  Verfahren nach Anspruch 4, worin die starke Säure aus der Gruppe bestehend aus Chlorwasserstoffsäure, Schwefelsäure und p-Toluolsulfonsäure ausgewählt ist.

7.  Verfahren nach Anspruch 3, welches ferner die Kondensation von 1,8-Naphthalindiamin mit einem Keton zur Gewinnung von 2,3-Dihydro-2,2-dialkylperimidin umfaßt.

8.  Verfahren nach Anspruch 7, worin:
    (i) das Alkylierungsmittel die Formel R-X aufweist, worin R $C_{1-10}$-Alkyl und X Br, I, eine Tosylatgruppe oder eine Mesylatgruppe bedeutet,
    (ii) das wassermischbare organische Lösungsmittel ein Keton ist, und
    (iii) die anorganische Base aus der Gruppe bestehend aus Kaliumcarbonat, Natriumcarbonat, Natriumhydroxid und Kaliumhydroxid ausgewählt ist.

9.  Verfahren nach Anspruch 7, worin die starke Säure aus der Gruppe bestehend aus Chlorwasserstoffsäure, Schwefelsäure und p-Toluolsulfonsäure ausgewählt ist.

10. Verfahren nach Anspruch 8, worin die starke Säure aus der Gruppe bestehend aus Chlorwasserstoffsäure, Schwefelsäure und p-Toluolsulfonsäure ausgewählt ist.

**Revendications**

1. Un procédé pour préparer une N,N'-dialkyl(en $C_1$ à $C_{20}$)-1,8-naphtalène diamine, selon lequel on hydrolyse une 2,3-dihydro-1,3-dialkyl (en $C_1$ à $C_{20}$)-2,2-dialkyl périmidine avec un acide aqueux fort dans un solvant organique aqueux et on élimine un sous-produit cétonique et le solvant organique aqueux par co-distillation.

2. Un procédé tel que revendiqué dans la revendication 1, dans lequel l'acide fort est choisi dans le groupe constitué de l'acide chlorhydrique, de l'acide sulfurique et de l'acide p-toluène sulfonique.

3. Un procédé pour préparer une N,N'-dialkyl(en $C_1$ à $C_{20}$)-1,8-naphtalène diamine, selon lequel:
   (a) on alkyle une 2,3-dihydro-2,2-dialkyl périmidine avec un agent alkylant (en $C_1$ à $C_{20}$ dans un solvant organique miscible à l'eau de la température ambiante à 80°C en présence d'eau et d'une base inorganique afin d'obtenir une 2,3-dihydro-1,3-dialkyl(en $C_1$ à $C_{20}$)-2,2-dialkyl périmidine; et
   (b) on hydrolyse la 2,3-dihydro-1,3-dialkyl(en $C_1$ à $C_{20}$)-2,2-dialkyl périmidine avec un acide fort aqueux dans du solvant organique aqueux après quoi on élimine un sous-produit cétonique et le solvant organique aqueux par co-distillation.

4. Un procédé tel que revendiqué dans la revendication 3, dans lequel:
   (i) l'agent alkylant a la formule R-X dans laquelle R est un alkyle en $C_1$ à $C_{10}$ et X est Br, I, un groupe tosylate ou un groupe mésylate;
   (ii) le solvant organique miscible à l'eau est une cétone; et
   (iii) la base inorganique est choisie dans le groupe constitué du carbonate de potassium, du carbonate de sodium, de l'hydroxyde de sodium et de l'hydroxyde de potassium.

5. Un procédé tel que revendiqué dans la revendication 3, dans lequel l'acide fort est choisi dans le groupe constitué de l'acide chlorhydrique, de l'acide sulfurique et de l'acide p-toluène sulfonique.

6. Un procédé tel que revendiqué dans la revendication 4, dans lequel l'acide fort est choisi dans le groupe constitué par l'acide chlorhydrique, l'acide sulfurique et l'acide p-toluène sulfonique.

7. Un procédé tel que revendiqué dans la revendication 3, comportant en outre la condensation de la 1,8-naphtalène diamine avec une cétone pour obtenir une 2,3-dihydro-2,2-dialkyl périmidine.

8. Un procédé tel que revendiqué dans la revendication 7, dans lequel:
   (i) l'agent alkylant a la formule R-X dans laquelle R est un alkyle en $C_1$ à $C_{10}$ et X est Br, I, un groupe tosylate ou un groupe mésylate;
   (ii) le solvant organique miscible à l'eau est une cétone; et
   (iii) la base inorganique est choisie dans le groupe constitué du carbonate de potassium, du carbonate de sodium, de l'hydroxyde de sodium et de l'hydroxyde de potassium.

9. Un procédé tel que revendiqué dans la revendication 7, dans lequel l'acide fort est choisi dans le groupe constitué de l'acide chlorhydrique, de l'acide sulfurique et de l'acide p-toluène sulfonique.

10. Un procédé tel que revendiqué dans la revendication 8, dans lequel l'acide fort est choisi dans le groupe constitué par l'acide chlorhydrique, l'acide sulfurique et l'acide p-toluène sulfonique.

Figure 1 - Condensation of 1,8-naphthalene diamine with a ketone or aldehyde to form 2,3-dihydro-2,2-dialkyl perimidine.

R₁, R₂ = alkyl or H

Figure 2 - Condensation of N,N'-dialkyl-1,8-naphthalene diamine with aldehyde or ketone to form 2,3-dihydro-1,3-dialkyl perimidines

R = alkyl
R₁, R₂ = alkyl or H

**Figure 3 - Reduction of diacetamide of 1,8-naphthalene diamine to N,N'-diethyl-1,8-naphthalene diamine**

**Figure 4 - New reductive methylation of 2,3-dihydro-2,2-dialkyl perimidine.**

$R_1$, $R_2$ = alkyl or H

# Figure 5 - Route to N,N'-dialkyl-1,8-naphthalene diamines.

DMF = dimethylformamide

Figure 6 - New route to N,N'-dialkyl-1,8-naphthalene diamines, with subsequent condensation to N,N'-dialkyl-2,3-dihydro-2,2-dialkyl or 2-alkyl perimidine.

R, R' = alkyl or H

$R_3$-X = $C_1$-$C_{20}$ alkyl halide (iodide or bromide), tosylate or mesylate

$R_3$ = $C_1$-$C_{20}$ alkyl

$R_4$, $R_5$ = H, $C_1$-$C_{20}$ alkyl, or form a cycloaliphatic ring